Europäisches Patentamt

European Patent Office

Office européen des brevets

(19)

(11) Numéro de publication: **0 289 682**
**A1**

## (12) DEMANDE DE BREVET EUROPEEN

(21) Numéro de dépôt: 87401045.7

(22) Date de dépôt: 07.05.87

(51) Int. Cl.⁴: **A61B 5/02** , **A61B 5/08**

(43) Date de publication de la demande:
09.11.88 Bulletin 88/45

(84) Etats contractants désignés:
AT BE CH DE ES FR GB GR IT LI LU NL SE

(71) Demandeur: **GRADIENT "Association régiée par la loi de 1901" Centre de Recherche de Royallieu Bâtiment A - B.P. 233
Rue Personne de Roberval
F-60206 COMPIEGNE CEDEX(FR)**

(72) Inventeur: **Farges, Gilbert
Logement des Instituteurs Ecole Phileas Lebesgues
F-60200 Compiègne(FR)**

(74) Mandataire: **Mongrédien, André et al
c/o SOCIETE DE PROTECTION DES INVENTIONS 25, rue de Ponthieu
F-75008 Paris(FR)**

(54) Appareil de surveillance cardio-respiratoire.

(57) L'invention concerne un appareil de surveillance cardio-respiratoire.

L'appareil comprend un capteur (1) de potentiels électriques cardiaques et un détecteur (7) de pics d'amplitude dans les signaux de rythme cardiaque fournis par le détecteur (7). L'appareil comporte aussi des moyens (18) reliés à la sortie du détecteur (7) pour extraire du signal de rythme cardiaque fourni par le détecteur, un signal représentatif du rythme respiratoire.

L'appareil s'applique à la surveillance cardio-respiratoire.

FIG. 1

EP 0 289 682 A1

# APPAREIL DE SURVEILLANCE CARDIO-RESPIRATOIRE

La présente invention concerne un appareil de surveillance cardio-respiratoire. Elle s'applique à la surveillance du rythme cardio-respiratoire de tout individu, et notamment à la surveillance du rythme cardio-respiratoire d'un nourrisson, d'un sportif, de tout individu pouvant être sujet à un accident cardiaque ou respiratoire, ...etc.

On sait que tout individu pouvant présenter des risques d'insuffisance cardio-respiratoire doit être soumis à une surveillance quasi permanente du rythme cardiaque et du rythme respiratoire. La surveillance de ces rythmes est par exemple encore plus importante pour les nourrissons dont la mort subite inexpliquée devient actuellement l'une des causes les plus importantes de mortalité avant l'âge de 1 an. Cette surveillance ne doit pas être négligée, notamment chez les sportifs amateurs, professionnels ou occasionnels.

En ce qui concerne les nourrissons, des statistiques récentes montrent qu'en France, 20 à 30% des décès d'enfants âgés de 1 mois à 1 an, sont provoqués par une insuffisance cardio-respiratoire. Cette insuffisance provoque ainsi 2000 décès par an en France et 10000 décès par an aux Etats-Unis. Les causes pathologiques de cette insuffisance sont inconnues malgré des recherches importantes. La seule possibilité d'éviter ces décès est de surveiller en permanence le rythme cardio-respiratoire, notamment des nourrissons, pendant leur sommeil nocturne ou diurne ; c'est en effet pendant le sommeil que la mort subite des nourrissons intervient le plus souvent. Cette mort touche des enfants apparemment sains et en bonne santé, et n'ayant jamais donné d'inquiétudes pédiatriques. Pourtant, le geste sauveur à effectuer est simple : il suffit par exemple d'une forte stimulation mécanique entraînant le réveil de l'enfant, quand une insuffisance de rythme est détectée, pour que cet enfant retrouve le contrôle normal de ses fonctions physiologiques ; d'où l'intérêt d'un appareil de détection d'insuffisance cardio-respiratoire muni d'une alarme.

La population d'enfants à risque cardio-respiratoire est estimée de la façon suivante :

-les enfants ayant déjà eu un accident cardio-respiratoire et sauvés in extrémis,

-les frères et soeurs d'un enfant de moins d'un an ayant déjà eu un accident cardio-respiratoire,

-certains enfants prématurés.

Les enfants à risque, qui ne présentent aucun signe pathologique majeur, ne peuvent être hospitalisés pendant un an et sont donc surveillés à domicile par des appareils de surveillane cardio-respiratoire. On estime qu'un enfant sur vingt qui présente de tels risques est ainsi surveillé à domicile.

Cette faible proportion est due à plusieurs facteurs :

-les appareils de surveillance cardio-respiratoire qui sont fiables et qui sont actuellement disponibles sur le marché, sont très onéreux,

-les organismes d'assurance ou de santé ne prennent pas en charge systématiquement le coût d'achat et d'entretien de ces appareils, ce qui rend la situation difficile pour les équipes médicales ainsi que les associations qui s'occupent de ce problème. Le corps médical est *très réticent* vis-à-vis de la surveillance à domicile : en effet, les appareils existants sont volumineux, lourds, peu pratiques. Ils constituent une gêne psychologique pour le milieu familial qui les utilise. Ces remarques concernant les nourrissons sont également applicables aux individus pouvant être sujets à un accident cardiaque ou respiratoire. Les appareils de surveillance cardio-respiratoire sont onéreux et ne peuvent être confiés en permanence aux nombreux patients qui présentent des risques d'accident cardiaque ou respiratoire.

Enfin, pour tous les autres individus, qui semblent en bonne santé, et qui par exemple pratiquent un sport en amateur, la surveillance cardio-respiratoire peut être utile, notamment pendant l'effort. Les appareils de surveillance cardio-respiratoire actuellement connus sont trop encombrants et trop onéreux pour effectuer cette surveillance durant l'effort.

Les appareils de surveillance cardio-respiratoire connus, les plus perfectionnés, sont de types très variés. L'appareil le plus utilisé et le plus fiable comporte un capteur à trois électrodes qui sont fixées sur la cage thoracique de l'individu à surveiller. L'une des électrodes sert de référence de tension. Le rythme cardiaque est obtenu à partir des signaux des potentiels électriques du coeur, prélevés entre les deux autres électrodes, tandis que le rythme respiratoire est obtenu par détection de la variation d'amplitude d'un courant électrique de faible intensité et de fréquence élevée, injecté à travers la cage thoracique par ces mêmes électrodes. En effet, les mouvements respiratoires entraînent des changements d'impédance électrique interne de la cage thoracique dûs aux variations de volume des organes à l'intérieur de celle-ci et, selon la loi d'Ohm, si un courant constant est injecté à travers une impédance variable, la tension aux bornes de cette impédance varie également. Les signaux représentatifs du rythme respiratoire et du rythme cardiaque sont mélangés à la sortie du capteur ; des moyens de filtrage permettent d'extraire d'une part les signaux représentatifs du rythme cardiaque, et d'autre part les signaux repésentatifs du rythme respiratoire. Un analyseur de fréquence permet de déterminer la valeur de la fréquence des signaux représentatifs du rythme cardiaque (signaux impulsionnels). Cet analyseur de fréquence est relié à un détecteur de seuils qui permet de déclencher une alarme lorsque la fréquence des signaux de rythme cardiaque est en dehors de seuils prédéterminés de fréquence haute et basse.

Les signaux représentatifs du rythme respiratoire, obtenus à la sortie de moyens de filtrage, sont aussi appliqués à un détecteur qui déclenche l'alarme lorsque l'amplitude ou la fréquence des signaux de rythme respiratoire est en dehors d'une gamme de fréquences ou d'amplitudes prédéterminées.

Ce type de dispositif présente un inconvénient très important : lorsqu'un individu cesse de respirer, l'arrêt du coeur n'est pas simultané, si bien que la cage thoracique, qui est soumise aux pulsations du coeur subit ainsi des mouvements internes non négligeables ; ces mouvements cardiaques font varier l'impédance interne de la cage thoracique et donc l'amplitude de la tension représentative du rythme respiratoire perçue par les électrodes du capteur et il en résulte qu'à la sortie des moyens de filtrage, des signaux comparables à ceux du rythme respiratoire sont obtenus, alors que la respiration a complètement cessé. Il en résulte selon la position des électrodes et le réglage de la sensibilité, qu'en cas d'arrêt respiratoire, aucune alarme n'est déclenchée ; cette alarme n'est alors déclenchée que lorsque le coeur a preque cessé de battre ou subit un ralentissement très important, et donc souvent trop tardivement.

A cet inconvénient majeur de ce type de dispositif s'ajoute, comme indiqué plus haut, son prix très onéreux, son volume très important, ainsi que des réglages difficiles des seuils de sensibilité du rythme respiratoire.

La présente invention a pour but de remédier à ces inconvénients et notamment de réaliser un appareil de surveillance cardio-respiratoire dans lequel intervient un capteur utilisant également deux ou trois électrodes, mais dans lequel les signaux représentatifs du rythme respiratoire ne sont pas obtenus séparément des signaux représentatifs du rythme cardiaque, mais sont précisément extraits de ces signaux de rythme cardiaque.

Cette obtention de signaux de rythme respiratoire, à partir des signaux de rythme cardiaque a pour principal avantage, comme on le verra plus loin en détail, d'assurer une surveillance bien plus fiable, et notamment de provoquer le déclenchement d'une alarme même lorsque les mouvements respiratoires ayant cessé, le coeur continue à battre. Le dispositif de l'invention a aussi pour avantage d'être peu encombrant, portable et autonome, sans gêne pour l'individu qui le porte ; son prix de revient est très faible, et la sécurité électrique est bien supérieure car aucun courant n'est injecté dans l'organisme.

L'invention a pour objet un appareil de surveillance cardio-respiratoire, comprenant un capteur de potentiels électriques cardiaques, un détecteur relié à une sortie du capteur pour détecter des pics d'amplitude dans les signaux fournis par le capteur caractérisé en ce qu'il comprend en outre des moyens reliés à la sortie du détecteur de pics d'amplitude pour extraire du signal fourni par ce détecteur de pics d'amplitude, un signal représentatif du rythme respiratoire.

Selon une autre caractéristique, l'appareil comporte en outre un détecteur de seuil d'amplitude du signal de rythme respiratoire ou d'au moins un seuil de fréquence du signal de rythme respiratoire, relié à une sortie des moyens d'extraction du signal représentatif du rythme respiratoire, une sortie de ce détecteur de seuil d'amplitude ou de fréquence de rythme respiratoire étant reliée à une alarme pour la déclencher lorsque l'amplitude du signal représentatif du rythme respiratoire est en dehors d'une gamme d'amplitudes prédéterminées autour du seuil d'amplitude, et/ou lorsque la fréquence du signal de rythme respiratoire est en dehors d'une gamme de fréquences prédéterminées autour du seuil de fréquence, et/ou lorsque la valeur du produit de l'amplitude du signal de rythme respiratoire par la période de ce signal est en dehors d'une gamme de valeurs prédéterminées autour de la valeur représentative du seuil de ce produit.

Selon une autre caractéristique, les moyens d'extraction d'un signal représentatif du rythme respiratoire comportent un circuit échantillonneur-bloqueur relié à la sortie du détecteur de pics d'amplitude, et un filtre relié à une sortie du circuit échantillonneur-bloqueur, pour extraire la composante moyenne du signal fourni par le circuit échantillonneur-bloqueur, cette composante moyenne étant ledit signal représentatif du rythme respiratoire fourni par une sortie du filtre qui constitue la sortie des moyens d'extraction.

Selon une autre caractéristique, les moyens d'extraction d'un signal de rythme respiratoire, sont de type numérique.

Selon une autre caractéristique, l'appareil comporte en outre un analyseur de fréquences relié à la sortie du détecteur de pics d'amplitude et à l'alarme pour déterminer la fréquence des pics détectés, cette fréquence étant représentative du rythme cardiaque, un détecteur d'au moins un seuil de fréquence de rythme cardiaque, relié à une sortie de l'analyseur pour fournir un signal de déclenchement de l'alarme lorsque la fréquence déterminée par l'analyseur est en dehors d'une gamme de fréquence, prédéterminée autour dudit seuil de fréquence de rythme cardiaque.

Selon une autre caractéristique le détecteur de seuil d'amplitude ou de fréquences de rythme respiratoire comporte des moyens de temporisation du déclenchement de l'alarme.

Selon une autre caractéristique, l'analyseur de fréquence est de type numérique.

Selon une autre caractéristique le détecteur de seuil de fréquence de rythme cardiaque comporte des moyens de temporisation du déclenchement de l'alarme.

Selon une autre caractéristique, le capteur comporte au moins deux électrodes disposées préférentiellement au voisinage du coeur.

Selon une autre caractéristique, le capteur comporte une troisième électrode de référence.

Selon une autre caractéristique, le détecteur de pics d'amplitude est relié aux moyens d'extraction de signal de rythme respiratoire et/ou à l'analyseur de fréquence, par des lignes électriques conductrices ou par des moyens de transmission sans fil.

L'invention sera mieux comprise à l'aide de la description qui va suivre, donnée en référence aux dessins annexés dans lesquels :

- la figure 1 représente schématiquement un appareil de surveillance cardio-respiratoire, conforme à l'invention,

- la figure 2 représente schématiquement en (a) et (b) des diagrammes de différents signaux obtenus dans l'appareil de l'invention ; ces diagrammes permettent de mieux comprendre le fonctionnement de l'appareil.

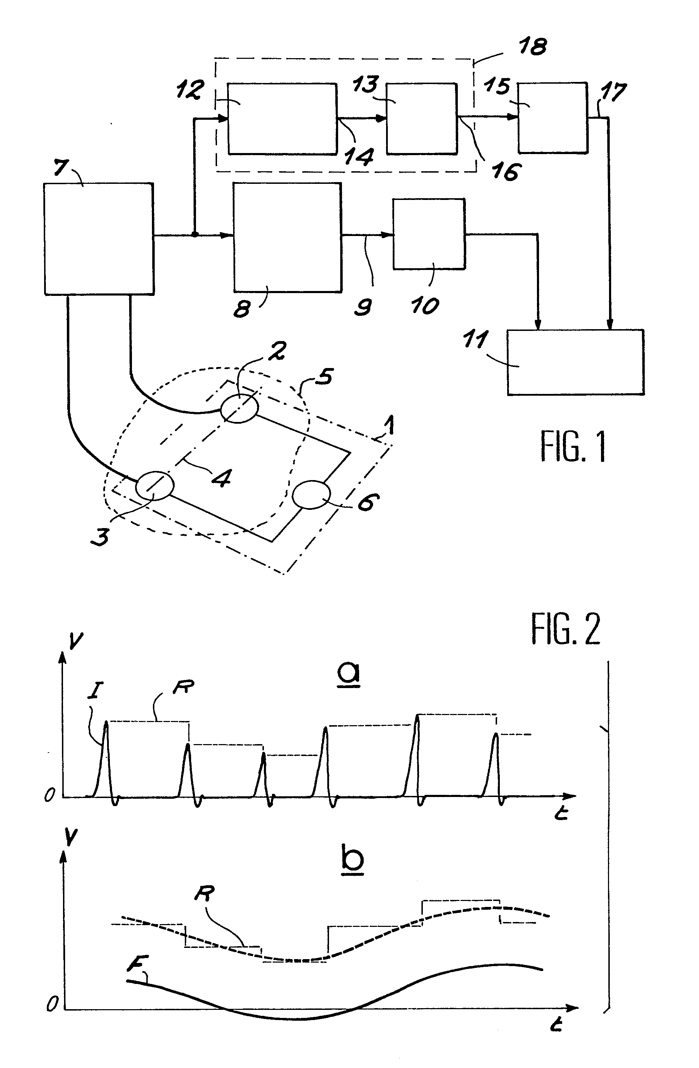

L'appareil représenté schématiquement sur la figure 1 comprend un capteur 1 de rythme cardiaque comprenant deux électrodes 2, 3 placées sur la poitrine de l'individu au voisinage du coeur 5 de préférence au voisinage de l'axe électrique 4 de celui-ci et une électrode de référence 6. De façon connue, les pulsations cardiaques provoquent entre les électrodes 2, 3 des variations périodiques de différence de potentiel. Ces variations périodiques sont des pics d'amplitude de tension apparaissant entre les deux électrodes 2, 3. La fréquence de ces pics de tension représente le rythme cardiaque. L'appareil comprend aussi un détecteur 7 de pics de tension qui permet de détecter les pics de tension dans les signaux fournis par le capteur. Cet appareil comprend aussi un analyseur de fréquence 8 pour déterminer la fréquence des signaux impulsionnels fournis par le détecteur 7. La fréquence de ces signaux est fournie sous forme analogique ou numérique, sur une sortie 9 de l'analyseur de fréquence 8. Cette sortie est reliée à l'entrée d'un détecteur 10 analogique ou numérique de seuil de fréquence des signaux impulsionnels représentatifs du rythme cardiaque. La sortie du détecteur 10 est reliée à une alarme 11. Le détecteur 10 peut comporter des moyens de temporisation du déclenchement de l'alarme 11 (non représentés sur la figure). Cette alarme est déclenchée lorsque la fréquence mesurée par l'analyseur 8 est en dehors d'une gamme de fréquences prédéterminées autour du seuil de fréquence des signaux de rythme cardiaque. En dehors de cette gamme, le rythme cardiaque est considéré comme anormal. Ces limites peuvent être fixées autour du seuil, soit par un circuit interne au détecteuur 10, soit par un circuit interne à l'alarme 11.

Selon l'invention, l'appareil comprend aussi des moyens 18 reliés au détecteur 7, pour extraire du signal fourni par le détecteur, un signal représentatif du rythme respiratoire.

Dans un mode de réalisation de type analogique, les moyens d'extraction 18 comprennent un circuit échantillonneur-bloqueur 12, relié à la sortie du détecteur 7 de pics d'amplitude des signaux de rythme cardiaque. Un filtre 13 est relié à une sortie 14 du circuit échantillonneur-bloqueur 12 pour extraire, comme on le verra plus loin en détail, la composante moyenne du signal fourni par ce circuit échantillonneur-bloqueur. Ce signal est représentatif du rythme respiratoire. Un détecteur de seuil 15 est relié à la sortie 16 du filtre 13. Une sortie 17 de ce détecteur 15 est reliée à l'alarme 11. Ce détecteur permet de déclencher l'alarme 11 lorsque l'amplitude du signal représentatif du rythme respiratoire est en dehors d'une gamme d'amplitudes prédéterminées autour d'un seuil prédéterminé d'amplitude, et/ou la fréquence du signal de rythme respiratoire est en dehors d'une gamme de fréquences prédéterminées autour d'un seuil prédéterminé de fréquence, et/ou lorsque la valeur du produit de l'amplitude du signal de rythme respiratoire par la période de ce signal est en dehors d'une gamme de valeurs prédéterminées autour d'une valeur de seuil prédéterminé de ce produit. Ces gammes de valeurs d'amplitudes, de fréquences ou de produits peuvent être fixées par un circuit interne au détecteur 15 ou à l'alarme 11.

Dans un mode de réalisation de type numérique, les moyens d'extraction 18 peuvent être constitués par un microprocesseur relié à une mémoire contenant un programme de traitement des signaux de rythme cardiaque fournis par le détecteur 7, préalablement convertis en signaux numériques, par un convertisseur d'entrée du microprocesseur. Ce programme de traitement n'est pas décrit ici en détail, mais il permet d'extraire des signaux de rythme cardiaque, un signal représentatif du rythme respiratoire, comme on le verra plus loin en détail.

Il faut aussi noter que les liaisons entre le détecteur 7 et les moyens d'extraction 18 et/ou entre ce détecteur et l'analyseur 8 de fréquences, peuvent être des liaisons par lignes électriques conductrices ou des moyens de transmission sans fil.

L'analyseur de fréquence 8, les détecteurs 10 et 17 et l'alarme 11 peuvent être de type analogique ou numérique. Enfin, des moyens d'enregistrement de type analogique ou numérique selon le mode de réalisation choisi, peuvent être connectés à la sortie de n'importe quel composant de l'appareil, pour permettre l'enregistrement et l'étude des signaux analogiques ou numériques fournis par chacun de ces composants.

La figure 2 représente schématiquement des diagrammes de certains signaux obtenus dans l'appareil de l'invention. Le diagramme (a) représente en I les signaux impulsionnels obtenus à la sortie du détecteur de pics 7. Ces signaux impulsionnels permettent de déterminer le rythme cardiaque puisque leur fréquence correspond à celle des pulsations du coeur. L'amplitude de chaque impulsion I dépend des différentes orientations du coeur lorsque celui-ci oscille dans la cage thoracique, par suite des mouvements respiratoires. Il en résulte que lorsque les mouvements respiratoires cessent et que le coeur continue à battre encore quelques minutes, l'amplitude des impulsions I devient constante puisque le coeur ne subit plus d'oscillation. La détection des variations d'amplitude des impulsions I, représentatives du rythme cardiaque, est donc très importante dans la détection du rythme respiratoire : le rythme cardiaque est déterminé comme on l'a indiqué plus haut, par un analyseur de fréquences et, lorsque ce rythme est situé en dehors d'une gamme de fréquences prédéterminées autour d'un seuil, l'alarme est déclenchée.

Le rythme respiratoire est déterminé de la façon suivante dans le mode de réalisation analogique : les signaux fournis par le détecteur de pics 7 sont appliqués à l'échantillonneur-bloqueur 12 commandé à l'instant du maximum d'amplitude ; cet échantillonneur-bloqueur permet d'obtenir sur une sortie 14, des signaux présentant des paliers, tels que représentés en R sur le diagramme (a) de la figure 2. Les détails de structure et de fontionnement de cet échantillonneur-bloqueur ne seront pas décrits ici en détal, puisque ce type de circuit est parfaitement connu dans l'état de la technique. Cet échantillonneur permet en fait d'obtenir des paliers de tension V entre deux impulsions successives I, par blocage de la tension lue en entrée au moment de l'échantillonnage, l'amplitude de la tension entre deux impulsions dépendant à la sortie de l'échantillonneur-bloqueur, de l'amplitude de l'impulsion précédente et de l'amplitude de l'impulsion suivante à chaque échantillonnage.

Les signaux R obtenus à la sortie 14 du circuit échantillonneur-bloqueur 12 sont appliqués à un filtre moyenneur 13 qui fournit sur sa sortie 16, un signal F de rythme respiratoire, tel que représenté sur le diagramme (b) de la figure 2. Ce signal F représente l'amplitude moyenne du signal de sortie de l'échantillonneur-bloqueur autour du zéro de tension. Quand un arrêt respiratoire se produit, le signal F devient plat, égal à zéro, et ne subit aucune perturbation trompeuse de la part du coeur, contrairement aux appareils connus de surveillance cardio-respiratoire. Les signaux R et F ont même fréquence et même période.

Le détecteur de seuils 15 qui est relié à la sortie 16 du filtre 13, déclenche l'alarme 11 dans les conditions indiquées plus haut. Ce détecteur de seuils, qui n'est pas décrit ici en détail, peut être constitué, de manière connue par un circuit de type trigger de Schmitt relié à la sortie du filtre 13, et par un circuit temporisateur relié à une sortie du trigger. Le circuit temporisateur fournit sur la sortie 17 un signal de niveau logique 1, par exemple lorsque le trigger de Schmitt fournit en permanence des impulsions résultant des variations d'amplitude du signal F ; ce circuit temporisateur fournit un signal de niveau logique 0 lorsque le trigger de Schmitt cesse de fournir des impulsions, c'est-à-dire lorsque le signal F cesse de présenter des variations d'amplitude suffisantes. Ce signal de niveau logique 0 permet de déclencher l'alarme 11. Dans le cas de détection de fréquences du signal représentant le rythme respiratoie, le trigger de Schmitt est associé à un détecteur de seuil de fréquence.

Dans le cas de détection de valeurs du produit de l'amplitude du signal de rythme respiratoire, par la période de ce signal, le trigger de Schmitt est associé à un circuit multiplicateur et à un détecteur de seuil de la valeur de ce produit.

Dans un mode de réalisation numérique de l'appareil, le circuit d'extraction 18 qui comporte un microprocesseur de traitement, fournit directement un signal numérique équivalent au signal F ou R. Les seuils d'amplitude ou de fréquence ou de valeurs de produits amplitude-fréquence, sont alors déterminés numériquement.

L'appareil qui vient d'être décrit permet bien de remédier aux inconvénients des appareils connus : l'alarme est déclenchée, sans ambiguïté soit parce que le rythme cardiaque est en dehors des limites prédéterminées de fréquences, soit parce que l'amplitude du signal de rythme respiratoire est en dehors de limites prédéterminées pendant une période trop importante, soit encore parce que la valeur du produit de l'amplitude du signal de rythme respiratoire par la période de ce signal, est en dehors de limites prédéterminées. Ce produit représente en effet la capacité ventilatoire de l'individu. Ces insuffisances ou anomalies peuvent d'ailleurs survenir simultanément et dans ce cas le détecteur de seuils 15 est conçu de façon appropriée.

L'appareil est autonome et peut être transporté par l'individu sous surveillance. Il peut être alimenté paar piles et son coût de fabrication est très faible.

En ce qui concerne l'amplitude du signal de rythme respiratoire, c'est généralement quand cette amplitude devient inférieure à un seuil prédéterminé, que l'alarme doit être déclenchée. Dans certains cas d'insuffisance respiratoire, pour lesquels le patient insuffisamment ventilé inspire très fortement, il peut être utile de déclencher également l'alarme lorsque l'amplitude du signal de rythme respiratoire devient trop importante.

On peut également fixer deux seuils de fréquences de signal de rythme cardiaque, en dehors desquelles l'alarme est déclenchée. Le seuil de fréquence basse correspond à une trop faible fréquence du coeur (bradycardie), tandis que le seuil de fréquence haute correspond à une trop grande fréquence du coeur (tachycardie).

**Revendications**

1. Appareil de surveillance cardio-respiratoire, comprenant un capteur (1) de potentiels électriques cardiaques, un détecteur (7) relié à une sortie du capteur pour détecter des pics d'amplitude dans les signaux de rythme cardiaque fournis par le capteur (1), caractérisé en ce qu'il comprend en outre des moyens (18) reliés à la sortie du détecteur (7) de pics d'amplitude pour extraire du signal fourni par ce détecteur de pics d'amplitude, un signal représentatif du rythme respiratoire.

2. Appareil selon la revendication 1, caractérisé en ce qu'il comporte en outre un détecteur (15) d'au moins un seuil prédéterminé d'amplitude du signal de rythme respiratoire et/ou d'au moins un seuil prédéterminé de fréquence du signal de rythme respiratoire et/ou d'au moins un seuil de valeur prédéterminée représentative du produit de l'amplitude du signal de rythme respiratoire par la période de ce signal, ce détecteur étant relié à une sortie des moyens d'extraction (18) du signal représentatif du ryhtme respiratoire, une sortie de ce détecteur (15) de seuil étant reliée à une alarme (11) pour la déclencher lorsque l'amplitude du signal représentatif du rythme respiratoire est en dehors d'une gamme d'amplitudes prédéterminées autour du seuil d'amplitude, et/ou lorsque la fréquence du signal de rythme respiratoire est en dehors d'une gamme de fréquences prédéterminées autour du seuil de fréquence, et/ou lorsque la valeur du produit de l'amplitude du signal de rythme respiratoire par la période de ce signal est en dehors d'une gamme de valeurs prédéterminées autour de la valeur de seuil prédéterminée de ce produit.

3. Appareil selon la revendication 1, caractérisé en ce que les moyens (18) d'extraction d'un signal représentatif du rythme respiratoire comportent un circuit échantillonneur-bloqueur (12) relié à la sortie du détecteur (7) et de pics d'amplitude, et un filtre (13) relié à une sortie du circuit échantillonneur-bloqueur (12), pour extraire la composante moyenne du signal fourni par le circuit échantillonneur-bloqueur, cette composante moyenne étant ledit signal représentatif du rythme respiratoire fourni par une sortie du filtre qui constitue la sortie des moyens d'extraction.

4. Appareil selon la revendication 1, caractérisé en ce que les moyens (18) d'extraction d'un signal de rythme respiratoire sont de type numérique.

5. Appareil selon l'une quelconque des revendications 1 à 4, caractérisé par le fait qu'il comporte en outre un analyseur de fréquences (8) relié à la sortie du détecteur (7) de pics d'amplitude et à l'alarme (11) pour déterminer la fréquence des pics détectés, cette fréquence étant représentative du rythme cardiaque, un détecteur (10) d'au moins un seuil prédéterminé de fréquence de rythme cardiaque, relié à une sortie de l'analyseur pour fournir un signal de déclenchement de l'alarme lorsque la fréquence déterminée par l'analyseur est en dehors d'une gamme de fréquences prédéterminées autour du seuil de fréquence de rythme cardiaque.

6. Appareil selon la revendication 5, caractérisé en ce que l'analyseur de fréquences est de type numérique.

7. Appareil selon la revendication 1, caractérisé en ce que le détecteur (15) de seuils d'amplitude ou de fréquences de rythme respiratoire comporte des moyens de temporisation du déclenchement de l'alarme (11).

8. Appareil selon la revendication 5, caractérisé en ce que le détecteur (10) de seuil de fréquence de rythme cardiaque comporte des moyens de temporisation du déclenchement de l'alarme (11).

9. Appareil selon la revendication 1, caractérisé en ce que le capteur (1) est un capteur à au moins deux électrodes disposées préférentiellement au voisinage du coeur.

10. Appareil selon la revendication 9, caractérisé en ce que le capteur (1) comporte une troisième électrode établissant un potentiel de référence.

11. Appareil selon la revendication 1, caractérisé en ce que le détecteur (7) de pics d'amplitude est relié aux moyens (18) d'extraction de signal de rythme respiratoire et/ou à l'analyseur de fréquence, par des lignes électriques conductrices ou par des moyens de transmission sans fil.

FIG. 1

FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A-2 017 312  (RIJKSUNIVERSITEIT TE GRONINGEN)<br>* page 1, ligne 67 - page 2, ligne 8; figure * | 1,3,5, 11 | A 61 B   5/02<br>A 61 B   5/08 |
| A | US-A-3 572 317  (WADE)<br>* colonne 2, ligne 68 - colonne 3, ligne 5; colonne 3, ligne 60 - colonne 8, ligne 18; figures 2, 3, 5 * | 1,2,5,7 ,9,10 | |
| A | WO-A-8 401 705  (THE LONDON HOSPITAL MEDICAL COLLEGE)<br>* page 4, ligne 24 - page 7, ligne 12 * | 1,2 | |
| A | WO-A-8 406 604  (AMERICAN TELEPHONE & TELEGRAPH CO.)<br>* résumé, figure 1 * | 1,2,4,6 ,9,10 | |

**DOMAINES TECHNIQUES RECHERCHES (Int. Cl.4)**

A 61 B   5/00

**Le présent rapport a été établi pour toutes les revendications**

| Lieu de la recherche | Date d'achèvement de la recherche | Examinateur |
|---|---|---|
| BERLIN | 08-12-1987 | WEIHS J.A. |

EPO FORM 1503 03.82 (P0402)